Europäisches Patentamt

European Patent Office

Office européen des brevets

(11) Publication number: **0 223 328 B1**

(12) **EUROPEAN PATENT SPECIFICATION**

(45) Date of publication of patent specification: **29.01.92**

(51) Int. Cl.⁵: **C07D 207/273**, A61K 31/40

(21) Application number: **86305607.3**

(22) Date of filing: **22.07.86**

(54) Process for producing oxiracetam.

(30) Priority: **26.07.85 JP 164019/85**
**07.02.86 JP 23915/86**

(43) Date of publication of application:
**27.05.87 Bulletin 87/22**

(45) Publication of the grant of the patent:
**29.01.92 Bulletin 92/05**

(84) Designated Contracting States:
**CH DE FR GB IT LI NL**

(56) References cited:
**EP-A- 0 046 274**
**EP-A- 0 156 655**
**CA-A- 1 059 137**
**FR-A- 2 380 257**
**US-A- 4 118 396**

(73) Proprietor: **DENKI KAGAKU KOGYO KABUSHIKI KAISHA**
**4-1, Yuraku-cho 1-chome**
**Chiyoda-ku Tokyo(JP)**

(72) Inventor: **Iriuchijima, Shinobu**
**Chuo Kenkyusho nai 5-1 Asahi-machi,**
**3-chome**
**Machida-shi Tokyo(JP)**
Inventor: **Kobayahi, Hirohiko**
**Chuo Kenkyusho nai 5-1 Asahi-machi,**
**3-chome**
**Machida-shi Tokyo(JP)**
Inventor: **Aoki, Kyoji**
**Chuo Kenkyusho nai 5-1 Asahi-machi,**
**3-chome**
**Machida-shi Tokyo(JP)**
Inventor: **Oda, Takeshi**
**Chuo Kenkyusho nai 5-1 Asahi-machi,**
**3-chome**
**Machida-shi Tokyo(JP)**
Inventor: **Shinoyama, Masayuki**
**Omi Kojo nai 2209 Oaza Omi Omi-machi**
**Nishi-kubiki-gun Niigata(JP)**
Inventor: **Nosaka, Yoshifumi**
**Omi Kojo nai 2209 Oaza Omi Omi-machi**
**Nishi-kubiki-gun Niigata(JP)**

(74) Representative: **Perry, Robert Edward et al**
**GILL JENNINGS & EVERY 53-64 Chancery Lane**
**London WC2A 1HN(GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to a process for producing oxiracetam (4-hydroxy-2-oxo-1-pyrrolidineacetamide) which is known as a pharmaceutical agent, for improving brain metabolism.

Various processes for producing oxiracetam are known. US-A-4118396 discloses obtaining it in five steps, using ethyl iminodiacetate and ethoxycarbonylacetyl chloride as raw materials. IT-A-0020227/1977 discloses obtaining it by reacting $\gamma$-amino-$\beta$-hydroxybutyric acid with hexamethyldisilazane, followed by a three-step reaction. IT-A-0019802/1984 discloses reacting a glycinamide derivative having a shielding group with a 3,4-epoxybutyric acid ester, followed by shield-removal and cyclisation. IT-A-0020358/1984 discloses using diketene as starting material, followed by reduction, shielding of the hydroxyl group, cyclisation, shield-removal and amidation.

CA-A-1059137 discloses the preparation of piracetam (2-oxo-1-pyrrolidineacetamide) by heating an amino-acid ester of the formula $H_2N\text{-}CO\text{-}CH_2\text{-}NH\text{-}(CH_2)_3\text{-}COOC_2H_5$.

EP-A-0156655 discloses producing oxiracetam by protecting the hydroxy group of a compound of the formula $HalCH_2\text{-}CHOH\text{-}CH_2\text{-}CO\text{-}NH\text{-}CH_2\text{-}COY$ (Y being $NR^1R^2$ or $OR$), cyclising the product in the presence of a strong non-nucleophilic base, and removing the protecting group.

FR-A-2380257 discloses preparing oxiracetam by reacting $\gamma$-amino-$\beta$-hydroxybutyric acid, under anhydrous conditions, with a silylating agent, with heating, to cause cyclisation, followed by reaction in the presence of an alkali metal hydride with a compound of the formula $Hal\text{-}(CH_2)_n\text{-}COOR$, and hydrolysing the resultant silyl derivative.

US-A-4118396 claims oxiracetam per se, and describes its preparation by subjecting the corresponding 3-carboxyl-substituted pyrroline to decarboxylation, followed by hydrogenation and ammonolysis.

Known processes for the preparation of oxiracetam have drawbacks such as expensive raw materials and multi-step reactions.

According to one aspect of the present invention, a process for producing oxiracetam comprises reacting glycine amide with a $C_{1-4}$ alkyl ester of a 4-halo-3-hydroxy-butanoic acid at 60 to 160°C, in the presence of a protonic solvent and of a base as a neutralising agent for hydrogen halide generated in the reaction.

According to a second aspect of the invention, a process for producing oxiracetam comprises reacting glycine amide with a $C_{1-4}$ alkyl ester of 3,4-epoxybutanoic acid at 60 to 160°C, in the presence of a protonic solvent.

Either of these processes produces oxiracetam in a single step.

The alkyl ester is, for example, a methyl, ethyl, propyl, butyl or isobutyl ester.

According to the first aspect of the invention, the starting material is a 4-halo-3-hydroxybutanoic acid ester. By way of example, 4-chloro-3-hydroxybutanoic acid alkyl esters are easily prepared, e.g. by carboxylation of epichlorohydrin which is commercially-available, at low cost (see Japanese Patent Application laid-open No. Sho 56-68644/1981). Further, glycine amide and its salts may be obtained by the reaction of ethyl monochloroacetate with aqueous ammonia (Z. Physiol. Chem. 64 348 (1910)).

An acid salt of glycine amide may be used to generate glycine amide in situ, by reaction with a base. The salt is, for example, the hydrochloride or sulphate. Suitable bases are metal alkoxides such as sodium ethoxide, alkali metal or alkaline earth metal hydroxides such as NaOH, KOH or Ca(OH)$_2$, and alkali metal carbonates such as Na$_2$CO$_3$, NaHCO$_3$ or K$_2$CO$_3$; in consideration of yield and economy, alkali metal carbonates are preferred.

Suitable protonic solvents include water, methanol, ethanol, propanol, butanol and 2-methoxyethanol; ethanol is preferred.

As the reaction proceeds, hydrogen halide is formed and, since this hydrogen halide hinders the reaction, a neutralising agent is present. As the neutralising agent, organic or inorganic bases are used. Examples of suitable organic bases are amines such as trimethylamine, N,N-diethylaniline and glycinamide; examples of suitable inorganic bases are alkali metal or alkaline earth metal hydroxides such as NaOH, KOH and Ca(OH)$_2$, and alkali metal carbonates such as Na$_2$CO$_3$, NaHCO$_3$ and K$_2$CO$_3$: in consideration of yield and economy, alkali metal carbonates are preferred, and Na$_2$CO$_3$ is particular preferred. The quantity thereof that is used is preferably one equivalent relative to the 4-halo-3-hydroxybutyric acid ester, and when glycine amide is used in the form of an acid salt such as the hydrochloride, it is preferred to add a further equivalent.

The reaction temperature is in the range of 60 to 160°C, preferably 70 to 130°C, and the reaction time is in the range of 1 to 70 hours, depending on the temperature and other conditions. In addition, a catalytic quantity of an iodide such as KI or NaI, promotes the present reaction.

According to the second aspect of the invention, the ester is of 3,4-epoxybutanoic acid, and the starting material is easily obtained, e.g. by (1) the dehydrochlorination reaction of 4-chloro-3-hydroxybutyric acid ester (J. Org. Chem. 32 3888 (1967)) or (2) epoxidation of 3-butenoic acid ester (Japanese Patent Application laid-open No. Sho 60-208957/1985).

The quantity of glycine amide used is preferably 1 to 1.5 equivalents per equivalent of the 3,4-epoxybutanoic acid ester. The conditions for carrying out the above reaction, with respect to solvent, reaction temperature and reaction time, are the same as those in the case of the 4-halo-3-hydroxybutanoic acid alkyl ester.

The process of the invention makes it possible to produce oxiracetam from inexpensive readily available raw materials.

Now, the present invention will be described more specifically below with reference to working examples.

Example 1:

In 10 mℓ of ethanol, 1.11g (10 m.moles) of glycinamide hydrochloride, 1.06 g (10 m.moles) of sodium carbonate, and 1.53 g (10 m.moles) of methyl 4-chloro-3-hydroxybutyrate were stirred and refluxed simultaneously for 20 hours. After completion of the reaction, the warm reaction mixture was filtered to expel inorganic salts. The filtrate was analyzed by gas chromatography (Fluoxylate-K 1% Uniport HP 100/120, column length 0.5 m and column temperature $220^\circ$C, $R_T$ 2.8min) (hereinafter referred to as "GC" for short). Consequently, the reaction was found to have produced oxiracetam in a yield of 75%. The filtrate was concentrated, dissolved in a small amount of water, and poured on a bed of 40 mℓ of Amberlite IR-120 ($-SO_3H$ form) (tradename: Rohm & Haas Co.). The adsorbate was eluted with water. The eluate was fractionated, with the first and second fractions discarded and the third and following fractions were collected. The collected fractions were concentrated. The concentrate was dissolved in methanol and the solution was ice cooled and crystallized with crystals of oxiracetam used as mother crystals. The produced crystals were collected and vaccum dried, to afford 0.55 g (yield of isolation 35%) of oxiracetam, having a melting point of $155^\circ$ to $160^\circ$C (as compared with $161^\circ$ to $163^\circ$C reported in the specification of Japanese Patent Publication Sho 58-22,034/1983).

Nuclear magnetic resonance (NMR) spectrum ($CD_3SOCD_3$), $\delta$ : 2.33 (2H, AB part of ABX system, J = 3, 6, 17 Hz), 3.43 (2H, AB part of ABX system, J = 2, 5.5, 10 Hz), 3.83 (2H, ABq, J = 17 Hz), 4.34 (1H, m), 5.20 (1H, br s), 7.13 (1H, br s), 7.30 (1H, br s).

Example 2:

In 10 mℓ of ethanol, 1.11 g (10 m.moles) of glycinamide hydrochloride, 1.06 g (10 m.moles) of sodium carbonate, and 1.53 g (10 m.moles) of methyl 4-chloro-3-hydroxybutyrate were stirred and refluxed simultaneously for 24 hours. The samples of resultant reaction mixture, taken 5 hours and 24 hours respectively after start of the reaction, were analyzed by GC for oxiracetam. Consequently, the yields were found to be 0% and 74% respectively.

Example 3:

The same raw materials as used in Example 2 plus 50 mg of potassium iodide were stirred and refluxed simultaneously for 24 hours by following the procedure of Example 2. The samples of resultant reaction mixture, taken 4 hours, 8 hours, and 24 hours respectively after start of the reaction, were analyzed by GC for oxiracetam. Consequently, the yields were found to be 27%, 47%, and 73% respectively.

Example 4:

The same raw materials as used in Example 2 plus 50 mg of sodium iodide were stirred and refluxed simultaneously for 24 hours by following the procedure of Example 2. The samples of resultant reaction mixture, taken 5 hours and 24 hours respectively after start of the reaction, were analyzed by GC for oxiracetam. Consequently, the yields were found to be 15% and 65% respectively.

Example 5:

In 10 mℓ of water, 1.11 g (10 m.moles) of glycinamide hydrochloride, 1.06 g (10 m.moles) of sodium carbonate, and 1.53 g (10 m.moles) of methyl 4-chloro-3-hydroxybutyrate were stirred at $80^\circ$C for 3 hours. After completion of the reaction, the resultant reaction mixture was analyzed by GC for oxiracetam. Consequently, the yield was found to be 41%.

Example 6:

In 10 mℓ of methanol, 1.11 g (10 m.moles) of glycinamide hydrochloride, 1.06 g (10 m.moles) of sodium carbonate, and 1.53 g (10 m.moles) of methyl 4-chloro-3-hydroxybutyrate were stirred and refluxed simultaneously for 20 hours. After completion of the reaction, the resultant reaction mixture was analyzed for oxiracetam. Consequently, the yield was found to be 45%.

Example 7:

In 10 mℓ of ethanol, 1.11 g (10 m.moles) of glycinamide hydrochloride, 1.68 g (20 m.moles) of sodium hydrogen carbonate, and 1.53 g (10 m.moles) of methyl 4-chloro-3-hydroxybutyrate were stirred and refluxed simultaneously for 21 hours. After completion of the reaction, the resultant reaction mixture was analyzed for oxiracetam. Consequently, the yield was found to be 65%.

Example 8:

In 10 mℓ of ethanol, 1.11 g (10 m.moles) of glycinamide hydrochloride, 1.38 g (10 m.moles) of potassium carbonate, and 1.53 g (10 m.moles) of methyl 4-chloro-3-hydroxybutyrate were stirred and refluxed simultaneously for 23 hours. After completion of the reaction, the resultant reaction mixture was analyzed for oxiracetam. Consequently, the yield was found to be 40%.

Example 9:

In 10 mℓ of ethanol, 1.11 g (10 m.moles) of glycinamide hydrochloride, 1.06 g (10 m.moles) of sodium carbonate, and 1.67 g (10 m.moles) of ethyl 4-chloro-3-hydroxybutyrate were stirred and refluxed simultaneously for 24 hours. After completion of the reaction, the resultant reaction mixture was analyzed for oxiracetam. Consequently, the yield was found to be 64%.

Example 10:

In 10 mℓ of 1N ethanol solution of sodium ethoxide, 1.11 g (10 m.moles) of glycinamide hydrochloride was stirred at room temperature for 1 hour. In the resultant solution, 0.53 g (5 m.moles) of sodium carbonate and 1.53 g (10 m.moles) of methyl 4-chloro-3-hydroxybutyrate were refluxed for 20 hours. After completion of the reaction, the resultant reaction mixture was analyzed for oxiracetam. The yield was found to be 65%.

Example 11:

In 10 mℓ of 1N ethanol solution of sodium hydroxide, 1.11 g (10 m.moles) of glycinamide hydrochloride was stirred at room temperature for 1 hour. In the resultant solution, 0.53 g (5 m.moles) of sodium carbonate and 1.53 g (10 m.moles) of methyl 4-chloro-3-hydroxybutyrate were refluxed for 20 hours. After completion of the reaction, the resultant reaction mixture was analyzed for oxiracetam. Consequently, the yield was found to be 60%.

Example 12:

The same raw materials as used in Example 2 were stirred at 70°C for 60 hours. After completion of the reaction, the resultant reaction mixture was analyzed for oxiracetam. Consequently, the yield was found to be 63%.

Example 13

Ethanol (10 mℓ) was added to glycinamide hydrochloride (1.11g, 10 m.moles), sodium hydrogen carbonate (0.84 g, 10 m.moles) and methyl 3,4-epoxybutyrate (1.16 g, 10 m.moles), followed by heating the mixture under reflux with stirring for 23 hours, filtering off inorganic salt after the reaction, and subjecting the filtrate to GC for determination. As a result, it was found that oxiracetam was formed with a yield of 50%.

The filtrate was concentrated and dissolved in a small quantity of water, followed by loading it on Amberlite IR-120 (-SO$_3$H type) (10 mℓ), eluting with water, washing the eluate with ethyl acetate, concentrating the aqueous layer, dissolving in methanol, ice cooling, crystallizing with a crystal matrix, collecting the resulting crystals, dissolving them in water, adding active carbon for decoloration, filtering off active carbon, concentrating the filtrate, dissolving the concentrate in a small quantity of water, adding acetone, ice-cooling for crystallization, and collecting and drying the resulting crystals to obtain oxiracetam (0.39 g, isolation yield 25%) in the form of white crystals.

M.P. 160-162°C (while 161-163°C according to Japanese patent publication No. Sho 58-22034/1983).

Nuclear Magnetic Resonance (NMR) spectrum (CD$_3$SOCD$_3$) $\delta$ : 2.33 (2H, AB part of ABX system, J = 3, 6,17 Hz), 3.43 (2H, AB part of ABX system, J = 2, 5.5, 10 Hz), 3.83 (2H, ABq, J = 17 Hz), 4.34 (1H, m), 5.20 (1H, br s), 7.13 (1H, br s), 7.30 (1H, br s).

Example 14

Ethanol (10 m) was added to glycinamide hydrochloride (1.11g, 10 m.moles), sodium carbonate (0.54 g, 5 m.moles) and methyl 3,4-epoxybutyrate (1.16 g, 10 m.moles), followed by refluxing the mixture with stirring for 20 hours, filtering off inorganic salt after the reaction, and determing the quantity of oxiracetam by means of GC. Yield: 35%.

Example 15

Ethanol (10 mℓ) was added to glycinamide hydrochloride (1.11 g, 10 m.moles), sodium hydrogen carbonate (0.84 g, 10 m.moles) and isobutyl 3,4-epoxybutyrate (1.58 g, 10 m.moles), followed by refluxing the mixture with stirring for 20 hours, filtering off after the reaction and determining the quantity of oxyracetam formed. Yield: 56%.

### Example 16

A 1N ethanol solution (10 mℓ) of sodium ethoxide was added to glycinamide hydrochloride (1.11 g, 10 m.moles), followed by stirring the mixture at room temperature for one hour, thereafter adding ethyl 3,4-epoxybutyrate (1.30 g, 10 m.moles), agitating and refluxing the mixture for 20 hours and after the reaction, determing the quantity of oxiracetam. Yield: 45%.

### Example 17

A 1N ethanol solution (10 mℓ) of sodium ethoxide was added to glycinamide semisulfate (1.23 g, 10 m.moles), followed by agitating the mixture at room temperature for one hour, thereafter adding methyl 3,4-epoxybutyrate (1.16 g, 10 m.moles), agitating and refluxing the mixture for 20 hours and after the reaction, determing the quantity of oxiracetam. Yield: 40%.

## Claims

1. A process for producing oxiracetam (4-hydroxy-2-oxo-1-pyrrolidineacetamide),which comprises reacting glycine amide with a $C_{1-4}$ alkyl ester of a 4-halo-3-hydroxybutanoic acid or of 3,4-epoxybutanoic acid in the presence of a protonic solvent, at 60 to 160°C, provided that, if the ester is of a 4-halo-3-hydroxybutanoic acid, the reaction is conducted in the presence of a base as a neutralising agent for hydrogen halide generated in the reaction.

2. A process according to claim 1, wherein the ester is of a 4-halo-3-hydroxybutanoic acid.

3. A process according to claim 2, wherein the ester is methyl 4-chloro-3-hydroxybutanoate.

4. A process according to claim 1, wherein the ester is methyl 3,4-epoxybutanoate or 2-methylpropyl 3,4-epoxybutanoate.

5. A process according to any preceding claim, wherein the glycine amide is formed in situ by reaction of an acid salt of glycine amide with a base.

6. A process according to any preceding claim, which is conducted in the presence of a catalyst.

7. A process according to claim 6, wherein the catalyst is a metal iodide.

## Revendications

1. Un procédé de production de l'oxiracétam (4-hydroxy-2-oxo-1-pyrrolidineacétamide), qui comprend la réaction du glycinamide avec un ester de $C_{1-4}$ alkyle d'un acide 4-halogéno-3-hydroxybutanoïque ou de l'acide 3,4-époxybutanoïque en présence d'un solvant protonique, à 60 à 160°C, à la condition que, lorsque l'ester est celui d'un acide 4-halogéno-3-hydroxybutanoïque, la réaction est conduite en présence d'une base en tant qu'agent de neutralisation de l'acide halogènhydrique généré dans la réaction.

2. Un procédé selon la revendication 1, selon lequel l'ester est celui d'un acide 4-halogéno-3-hydroxybutanoïque.

3. Un procédé selon la revendication 2, selon lequel l'ester est le 4-chloro-3-hydroxybutanoate de méthyle.

4. Un procédé selon la revendication 1, selon lequel l'ester est le 3,4-époxybutanoate de méthyle ou le 3,4-époxybutanoate de 2-méthylpropyle.

5. Un procédé selon l'une quelconque des revendications précédentes, selon lequel le glycinamide est formé in situ par réaction d'un sel acide de glycinamide avec une base.

6. Un procédé selon l'une quelconque des revendications précédentes, qui est conduit en présence d'un catalyseur.

7. Un procédé selon la revendication 6, selon lequel le catalyseur est un iodure métallique.

## Patentansprüche

1. Verfahren zur Herstellung von Oxiracetam (4-Hydroxy-2-oxo-1-pyrrolidinacetamid), bei dem Glycinamid mit einem $C_{1-4}$-Alkylester einer 4-Halogen-3-hydroxybutansäureoder einer 3,4-Epoxybutansäure in Gegenwart eines protonischen Lösungsmittels bei 60 bis 160°C umgesetzt wird, wobei dann, wenn es sich um den Ester einer 4-Halogen-3-hydroxybutansäure handelt, die Reaktion in Gegenwart einer Base als Neutralisationsmittel für den in der Reaktion gebildeten Halogenwasserstoff durchgeführt wird.

2. Verfahren nach Anspruch 1, wobei der Ester von einer 4-Halogen-3-hydroxybutansäure stammt.

3. Verfahren nach Anspruch 2, wobei der Ester Methyl-4-chlor-3-hydroxybutanoat ist.

4. Verfahren nach Anspruch 1, wobei der Ester Methyl-3,4-epoxybutanoat oder 2-Methylpropyl-3,4-epoxybutanoat ist.

5. Verfahren nach einem der vorhergehenden Patentansprüche, wobei das Glycinamid in situ durch Umsetzung eines Säuresalzes von Glycinamid mit einer Base gebildet wird.

6. Verfahren nach einem der vorhergehenden Patentansprüche, das in Gegenwart eines Katalysators durchgeführt wird.

7. Verfahren nach Anspruch 6, bei dem der Katalysator ein Metalliodid ist.